# EUROPEAN PATENT SPECIFICATION

(11) **EP 0 559 845 B1**
(45) Date of publication and mention of the grant of the patent: **17.12.1997**
(21) Application number: 92907412.8
(22) Date of filing: 01.04.1992
(51) Int. Cl.: A61K 31/70, C07H 19/167, G01N 33/92, A61K 31/00

(54) **Use of compounds in preventing endotoxin shock**
Verwendung von Substanzen zur Verhütung von Endotoxinschock
Utilisation des composés pour empecher le choc endotoxinique

(30) Priority: 05.04.1991 US 681036
(43) Date of publication of application: 15.09.1993
(73) Proprietor: WISCONSIN ALUMNI RESEARCH FOUNDATION, Madison, WI 53707-7365 (US)
(72) Inventor: PROCTOR, Richard, A., Madison, WI 53705 (US); BERTICS, Paul, J., Oregon, WI 53575 (US)
(74) Representative: Patentanwälte Ruff, Beier, Schöndorf und Mütschele
(86) International application number: US9202789
(87) International publication number: WO9217186

(56) References cited:
- US-A- 3 752 805
- US-A- 3 860 706
- US-A- 4 918 061
- US-A- 4 918 061
- J.CELL.BIOL. vol. 109, no. 4(2) , 1989 page 52A. 'Lipid X inhibition of endotoxin-stimulated GTPase activity in membranes from the macrophage cell line RAW 264.7'
- INT.CONGR.SER. - EXCERPTA MED. vol. 923 , 1990 pages 227 - 37 'Lipid X inhibition of bacterial lipopolysaccharide-stimulated GTPase activity'
- ZENTRALBL.BAKTERIOL., MIKROBIOL.HYG., ABT.1, SUPPL. vol. 15 , 1986 pages 33 - 39 'Photoaffinity labelling of botulinum neurotoxin by azido-ATP'
- Proc.Natl.Acad.Sci. USA, vol.91, June 1994, pages 6017-6020
- LEHNINGER, ALBERT, Biochemistry: The Molecular Basis of Cell Structure and Function, Worth Publishers.

## Description

This invention relates to the use of a compound, other than lipid X, which inhibits LPS-induced GTPase activity for the preparation of a pharmaceutical composition for treatment of mammals to prevent the deleterious effects of Gram-negative endotoxin (lipopolysaccharide or "LPS"). This invention also relates to an assay for identifying compounds which prevent endotoxin shock by measuring their ability to inhibit LPS-induced GTPase activity.

Endotoxin is a lipopolysaccharide (LPS) which is a major constituent of the outer-leaflet of the membranes of Gram-negative bacteria. Structural studies have shown that it consists of the following three distinct domains: 1) the 0-antigen region, which is a strain-specific polysaccharide moiety and determines the antigenic specificity of the organism; 2) the core region, which is relatively conserved with respect to its sugar composition and may play a role in maintaining the integrity of the outer membrane; and 3) the lipid A region, which is also conserved and functions as a hydrophobic anchor holding lipopolysaccharide in place. The lipid A portion of lipopolysaccharide constitutes most of the outer monolayer of the outer membrane in Gram-negatives.

Lipopolysaccharide is known to trigger many pathophysiological events in mammals, either when it is injected or when it accumulates due to Gram-negative infection. In general, the hycrophobic lipid A moiety is responsible for these pathophysiological effects, which tend to be either immunostimulatory or toxic. In the former category there are events such as B-lymphocyte mitogenesis, macrophage activation, and the induction of tumor necrosis in certain experimental systems. In the latter (toxic) category there are responses such as peripheral vascular collapse ("endotoxic" shock), pulmonary hypertension, pulmonary edema, disseminated intravascular coagulopathy and pyrogenicity.

The release of mediators such as tumor necrosis factor-α (TNF), interleukin-1 (IL-1), and interleukin-6 (IL-6) is thought to produce the toxicity associated with endotoxemia. However, despite the high mortality rate of endotoxic shock, relatively little is known about the biochemical mechanisms involved in LPS-induced events, e.g., TNF and IL-1 release, although an amplification system is suggested given that nanogram quantities or LPS can produce severe toxicity in animals. Most amplification pathways involve receptors and various enzyme cascades, thus allowing for several points of antagonism within the system. Although certain steps in LPS action are known, such as the stimulation of phosphoinositide hydrolysis, a transient increase in intracellular Ca⁺⁺ levels, and the activation of protein kinase C and phospholipase A₂, the lack of specific information on the cellular mechanisms involved in LPS action has impeded the development of therapeutic agents for preventing endotoxin shock.

The use of a compound named lipid X in the treatment of mammals is described in US-A-4918061. According to the disclosure of this patent, animals can be protected from the toxic effects of Gram-negative endotoxin by administering to these animals a safe and effective amount of the compound lipid X. Further, J.Cell.Biol., 109 (4 Pt.2). 1989. 52A. shows lipid X inhibition of endotoxin-stimulated GTPase activity in membranes from the macrophage cell line RAW 264.7 and Int. Congr.Ser. - Excerpta Med., 923, 1990, 227-37 describes lipid X inhibition of bacterial lipopolysaccharide-stimulated GTPase activity. According to all three publications, lipid X is defined as an endotoxin biosynthetic precursor and subunit. None of these publications does suggest that LPS-stimulated GTPase inhibitors other than lipid X can be used to prevent endotoxin toxicity. There is also no suggestion that any other inhibitors would prevent the detrimental effects of endotoxin mediators.

Obviously, it would be advantageous to have an assay for identifying compounds that could be useful as therapeutic agents in methods to prevent endotoxin shock. It also would be useful to have an assay to identify compounds which inhibit the release of tumor necrosis factor (TNF) and interleukin-1 (IL-1).

It is an object to disclose uses of compounds for treatment of mammals to protect them from the deleterious effects of Gram-negative endotoxins and to inhibit the release or TNF and IL-1, which comprise use of compounds (excluding lipid X) which have been identified by the assay of the present invention as inhibiting LPS-induced GTPase in vitro for the preparation of a pharmaceutical composition.

It is also an object of the present invention to disclose an assay for identifying new or known compounds which are useful to prevent endotoxin shock by measuring their ability to inhibit LPS-induced GTPase in vitro.

It is a further object to disclose an assay for identifying compounds that inhibit the release of endotoxic shock mediators, such are TNF, by measuring their ability to inhibit LPS-induced GTPase in vitro.

The assay of the present invention for identifying the compounds of interest basically comprises testing candidate compounds to determine if they are effective in blocking LPS-induced GTPase activity in macrophage membrances in vitro. We have discovered that compounds like 2-methylthio-ATP, which inhibit GTPase in the assay also prevent endotoxin shock or inhibit the release of TNF, a mediator of endotoxic shock.

The molecular mechanisms surrounding the toxicity and high mortality rate that accompanies the release or bacterial lipopolysaccharide (LPS) are unclear, although its potent activity suggests that an amplification system is involved. Because previous studies suggested that a G-protein (a GTP-binding protein that becomes active when compounds bind to receptor and cause the G-protein to activate other cellular effector molecules) may participate in LPS action, we evaluated the effects of LPS on GTPase activity in membranes isolated from a macrophage (RAW 264.7) cell line. (G-proteins cause hydrolysis of GTP, hence, they are GTPases. ) We found that LPS induced substantial GTPase activation (200-300% above basal), and kinetic analyses indicated that the maximal LPS-stimulated increase in velocity is observed within 15 min, that it is a low Kₘ (GTP) activity, that it can be enhanced by ammonium sulfate, and that it appears to be pertussis toxin-insensitive.

Moreover, we found the LPS-enhanced GTPase activity was not antagonized by phosphatase/ATPase inhibitors such as p-nitrophenyl-phosphate, ouabain, bafilomycin or N-ethyl-maleimide, and in fact was potentiated by the addition of ATP or ADP. We found a synergistic effect on GTPase activity when both LPS and ATP or ADP are added to the macrophage assay. These data suggest that LPS may provide a link between GTPase and a purinoreceptor.

We also found that the LPS precursor, lipid X, which can reduce the lethal effects of LPS endotoxin, caused a dose-dependent inhibition of the LPS-mediated stimulation of GTPase activity. Half-maximal inhibition was seen at the same ratio of lipid X to LPS known to be effective in preventing endotoxin effects in vivo, i.e. at a one to one weight ratio. These effects are specific because other phospholipids, detergents and glycosides neither stimulated basal GTPase activity nor inhibited LPS-induced GTPase activity. Our discovery that 2-methylthio-ATP blocks both LPS-induced GTPase activity and TNF production in RAW 2647 macrophages provides additional evidence that GTPase is a determinative pathway for mediating endotoxicity.

The uses of the present invention comprise preparation of a pharmaceutical composition for mammals to be protected from the deleterious effects of endotoxin (LPS) with compounds identified by the assay as inhibiting GTPase (with the exception of lipid X already known for such use). One such compound identified by the assay of the present invention is 2-methylthio-ATP.

The foregoing and further objects of the present invention will be apparent to those skilled in the art from the description.

We sought to directly evaluate the influence of LPS on possible G-protein function by examining agonist-enhanced GTPase activity, which is a hallmark of G-protein action. Moreover, because macrophages are one of the key cell types in the release of toxic mediators following an LPS challenge, we first assessed the capacity of LPS to influence GTPase activity in membranes isolated from RAW 264.7 cells, which is a highly LPS-responsive macrophage cell line. In these studies, GTPase activity was measured using a standard assay. The activity was determined using 2 µM γ-³²P-GTP in the presence of 0.1 mM ATP and 0.2 mM of the non-hydrolyzable ATP analog, AMPPNP, in order to minimize nonspecific nucleotidase activity in the membrane preparations. In the preferred practice of the present invention, the ability of a candidate compound to inhibit LPS-induced GTPase is assessed by measuring the release of ³²Pi from γ-³²P-GTP.

We first determined the optimal LPS concentration for GTPase stimulation. In all experiments, the LPS dose response curve was quite steep, and with the highest activity occurring around 200-300 µg LPS/ml. When the time course of LPS-activated GTPase was studied, LPS was found to enhance the rate of GTP hydrolysis as early as 2 min after addition, and the effect was maximal by 15 min.

### 1. GTPase Assay

The preferred assay is comparable to that described by Cassel et al., Biochem, Biophys. Acta 452, 538-551 (1976), and Neer et al., J. Biol. Chem. 259, 14222-14229 (1984), with two main exceptions: (1) We add ammonium sulfate because we found that although 300 µg LPS/ml increased GTPase activity by ∼ 30% in the absence of ammonium sulfate, in the presence of an optimal amount of ammonium sulfate (250 mM), the LPS-induced GTPase activity was nearly 3-fold above the reduced basal rate. Under these conditions, GTPase activation was evident at 100 µg LPS/ml (roughly 6 µM assuming an average **MW** ∼ 17,000 kDa), and the maximal effect was seen by 300 µg/ml LPS. (2) To show adenosine mucleotide synergism with LPS-stimulated GTPase activity, either 10µM ATP or 30µM ADP replace the 0.1 mM ATP and 0.2 mM AMPPNP of the standard assay described by Cassel et al. and Neer et al.

In the preferred assay, a candidate compound (.001 - 1000 µM) is dissolved or suspended in 100µl of a reaction buffer containing 20mM HEPES (pH 7.4), 0.01mM ATP or ADP, membranes isolated from RAW 264.7 cells, 2µM γ-³²P-GTP (3-9 X 10cpm/pmol), 5mM MgCl₂, 18µM LPS (based on an estimated MW of 17,000 kDa for E. coli 0111:B4 endotoxin), and 250 mM ammonium sulfate. The reaction is initiated by the addition of the γ-³²P-GTP and the mixture incubated at 30-37°C for 15 to 30 minutes. The reaction is terminated by the addition of 500µl of 5% trichloroacetic acid and 500µl of 0.1gm/ml acid-activated charcoal in 5% trichloroacetic acid. The samples are centrifuged at 14,000xg for 10 minutes, and a 550µl aliquot of the supernatant is removed for scintillation counting. The supernatant will contain the released ³²Pi. The extent of GTPase inhibition is measured by the decrease in the amount of ³²Pi released from γ-³²P-GTP. Appropriate controls include mixtures with one of the following omitted: (i) test compound, (ii) endotoxin, and (iii) ATP, ADP or AMPPNP.

The macrophage-like cell line RAW 264.7 is cultured as described using RPMI 1640 medium supplemented with 10% fetal calf serum (FCS) containing < 0.1 ng/ml LPS. The membranes are resuspended in 20 mM HEPES (pH 7.4), 1 mM dithiothreitol, 1 mM EDTA and 10 µg leupeptin/ml. Aliquots are stored at -70°C until assayed.

### 2. Effect of ATP

To further characterize the specificity of the LPS-stimulated GTPase, we first assayed its activity in the absence of any other nucleotides, and then compared this response to that found in reactions containing various ATPase/nucleotidase inhibitors. If the GTPase activity is due to a generalized increase in nucleotidase-like activities, one would expect that the inclusion of these inhibitors would attenuate the LPS-stimulated activity. Release of ³²Pi from γ-³²P-GTP would be predicted to be unaffected or even enhanced (more non-specific substrate hydrolysis) in the absence of these agents. Surprisingly, we observed that in the absence of any added adenine nucleotides, there was very little detectable LPS-enhanced GTPase activity. However, when either ATP, ADP or the non-hydrolyzable ATP analog AMPPNP was included in the incubation, there was a pronounced increase in LPS-stimulated GTPase activity. Moreover, the addition of the ATPase inhibitors ouabain, bafilomycin and N-ethyl-maleimide did not block LPS-stimulated GTPase activity in the presence of ADP. Conversely, as expected, the very general phosphoryl transferase inhibitor, sodium orthovanadate, did block LPS activation.

Because the above inhibitor studies indicated a maximum LPS-stimulated GTPase activity in the presence of ATP and ATP analogs, we investigated this interaction by measuring the ATP-dependence of the GTPase activity in macrophage membranes in the presence and absence of an optimal dose of LPS. ATP alone exhibited a biphasic stimulation of GTPase activity, with the maximum effect being observed around 1-10 µM. This stimulation by ATP may be due to the action of purinergic receptors, i.e., the adenosine and ATP receptors which are well-characterized to be coupled to various G-proteins.

When ATP and LPS are added together, there is a striking stimulation of GTPase activity at 1-10 µM ATP which is much greater than that seen with ATP or LPS alone. This synergistic stimulation by ATP plus LPS is also biphasic, with the inhibition of GTPase activity observed at very high ATP levels probably resulting from competitive or ionic effects. These data suggest that LPS may interact in some fashion with the purinergic receptor signal transduction pathway and that the LPS-mediated GTPase activation is not the result of a non-specific ATPase or other nucleotidase activity.

To assess whether the LPS/ATP-enhanced GTPase is a "low Kₘ" form, which would be expected for a variety of large and small MW G-proteins, as well as for various GAP and ARF-like proteins, as opposed to a "high Kₘ" form, such as a phosphatase, we assayed the GTPase activity using 0.2 and 50 µM GTP essentially as described previously. In view of the interactive effects of LPS and ATP, we measured high and low Kₘ GTPase in the absence of added ATP, as well as in the presence of a maximal activating dose of ATP (10 µM), and compared these results to those obtained using our standard assay conditions (2 µM GTP, 0.1 mM ATP, 0.2 mM AMPPNP). The LPS and ATP stimulated a low Kₘ GTPase activity by ∼30% and 70%, respectively, when added individually. This stimulation of low Kₘ activity was slightly greater than that estimated using the standard assay conditions. In addition, the combined effects of LPS and ATP on GTPase activity also appeared to represent an influence on a low Kₘ component, with the stimulation calculated for the low Kₘ activity (228% of basal activity) being comparable to the stimulation (208% of basal activity) measured using our standard assay conditions. In sum, the LPS-stimulated GTPase activity appears specific for GTP, it exhibits a low Kₘ (GTP), and it is insensitive to various ATPase inhibitors. The LPS stimulation of GTPase activity is enhanced by adenine nucleotides.

### 3. Effect of ATP Analogs

In the presence of LPS, ATP results in a three-fold increase in activity of the GTPase. Therefore, we examined the ability of ATP analogs to stimulate LPS-enhanced GTPase activity. In dose-response studies, the relative abilities of purines to stimulate LPS-enhanced GTPase were: ATP> ATPγS> ADP> AMPPNP>> β,γ methylene ATP> 2-MeSATP. The structure of 2-MeSATP is given below:

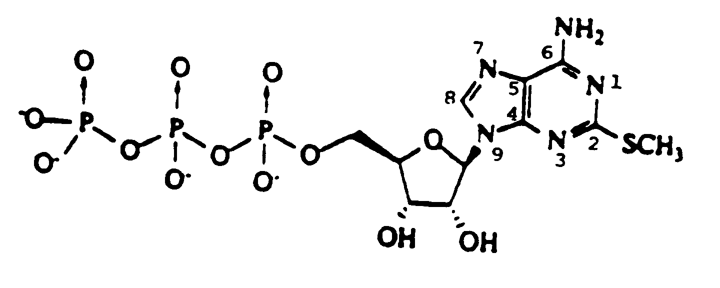

AMP and adenosine at 100 µM were ineffective, while ATP was active at 1 µM. This is the general order of purine agonist activity for the P2-type purinoreceptor (P2-R). Also, IBMX, an antagonist of both types of P1 purinoreceptors, but with no effect on P2 receptors, did not alter the LPS activation of the GTPase. (There are no known P2x, P2y, or P2z receptor antagonists currently available, while ATP is a P2t inhibitor, but an agonist in our system.) Of interest, one of the Gi-like proteins linked to the P2-purinoreceptors is resistant to pertussis toxin inactivation which is consistent with our results. Also, P2-receptor activation is linked to inositol phosphate breakdown in HL60 cells. Finally, LPS has been reported to decrease purine exonucleotidase activity of glomerular endothelial cells, as assessed by enzyme cytochemistry. These results open the possibility that the P2-R may be involved some in LPS-mediated activities. For example, since ATP infusion is known to result in shock, one might hypothesize the following events: LPS sensitizes P2-R to ATP via enhanced ligand binding to the P2-R or increased G-protein interaction with P2-R, and LPS might decrease intravascular breakdown ATP and ADP. Thus, in the presence of LPS, normal concentrations of ATP might result in shock and death.

Examination of the dose-response curves of ATP and ATP analogs, show that 2-methylthioATP (2-MeSATP) has no stimulatory effect on LPS-induced GTPase activity. Because 2-MeSATP is a potent agonist for two of the subtypes of the P2-R (P2y- and P2z-receptors), but showed little activity in our assay, we hypothesized that 2-MeSATP might be an antagonist. We found that 2-MeSATP did antagonize the stimulatory effects of ATP on LPS-inducible GTPase activity. When increasing amounts of 2-MeSATP (0-100µM) were added to the macrophage assay system, the activity of the LPS-enhanced GTPase decreased.

Moreover, 2-MeSATP decreased LPS-mediated TNFα production by RAW264.7 macrophages. Production of TNF by the macrophage cells was measured by a "cell viability" assay. This is a standard assay described in Birkland, et al., Adv. Exp. Med. Biol. 256: 399-402 (1990). In brief, TNF in solution will kill actinomycin D-sensitized L929 cells, which can be measured by a loss of MTT dye reduction. The supernatent was removed from RAW264.7 cells treated with 2-MeSATP, LPS and ATP. The greater the amount of 2-MeSATP added (0-5µM), the less TNF was produced by the macrophage cells.

### 4. Influence of Lipid X on LPS-Stimulable GTPase

The effectiveness of the GTPase assay to identify compounds that inhibit endotoxic shock was confirmed by using lipid X, a known endotoxin inhibitor. Because earlier work has suggested that lipid X can block LPS action in vivo, we performed experiments to determine whether lipid X could also antagonize LPS-stimulated GTPase activity in vitro. We found that lipid X was capable of dose-dependently inhibiting the LPS-enhanced GTPase activity. Using LPS concentrations of either 300 µg/ml or 1 mg/ml to enhance macrophage GTPase activity, lipid X exhibited half-maximal inhibition when the two agents were present at an ∼ 23 to 1 molar ratio of lipid X to LPS (1:1 weight ratio). This was consistent with our previous studies that found a similar ratio of lipid X to LPS to be half-maximally effective at blocking LPS shock in vivo, indicating that the relative concentrations of LPS and lipid X required for GTPase inhibition closely parallel that concentration needed for the antagonism of LPS toxicity under pathophysiological conditions.

We found that a synthetic lipid X derivative, bisphospho-aza-lipid X, was effective in blocking LPS-induced GTPase activity. It has the utility of being more water soluble. Although the major influence of lipid X appeared to be on the inhibition of LPS-stimulated GTPase activity, lipid X was also capable of slightly reducing basal activity suggesting a possible direct interaction at the GTPase level. Furthermore, with regard to the reversibility of the lipid X inhibition of LPS-stimulated GTPase activity, we observed this GTPase inhibition regardless of whether LPS or lipid X was first incubated with the membrane preparation.

### 5. Specificity of the LPS and Lipid X Effects

In view of the general lipophilicity of LPS and lipid X, other hydrophobic compounds were tested to demonstrate the specificity of the LPS and lipid X-mediated effects on GTPase activation/inhibition. We found that various phospholipids, such as phosphatidylcholine, phosphatidic acid and phosphatidylserine did not stimulate basal GTPase activity nor inhibit LPS-stimulated GTPase activity in macrophage membranes. The detergent Triton X-100 did reduce basal GTPase activity, but it did not substantially interfere with the LPS-stimulated activity. These data suggest that the effects of LPS and lipid X are not strictly due to general detergent or other lipid interactions.

With respect to non-LPS-stimulated GTPase activity in this macrophage system, we observed that 10% FCS (<0.1 ng endotoxin/ml serum by the Limulus amebocyte lysate method) induced a large degree of GTPase activation, whereas 3 mg/ml lipid X inhibited the FCS-enhanced GTPase activity by ∼ 50%. These results also suggest than lipid X may have direct effects, because it can apparently interfere with other agonist-enhanced GTPase activities.

Although these experiments were designed to minimize non-specific GTP hydrolysis by using very low GTP levels (2 µM) and very high concentrations of ATP/nucleotidase antagonists, i.e. 0.1 mM ATP and 0.2 mM AMPPNP, it was still possible that some of the observed effects were due to the activation of membrane phosphatases, e.g. alkaline phosphatase, or ATPases such as the abundant NaK-ATPase. However, the contribution of these enzymes may be discounted based on the fact that the LPS-stimulated GTPase activity exhibited an insensitivity to high concentrations of the phosphatase inhibitor, p-nitrophenyl-phosphate, and the ATPase inhibitor, ouabain, even in the presence of 0.1 mM ATP and 0.2 mM AMPPNP.

### 6. Animal Studies

### a. In General

In the preferred practice of the use of the present invention, a compound (not lipid X) identified by the assay as inhibiting GTPase activity is administered to an animal in a safe and effective amount to protect it from the toxic effects of Gram-negative endotoxin. The preferred compound is 2-methylthio-ATP (2-MeSATP) and the preferred route of administration is intravenously.

The need for additional compounds that will prevent endotoxin shock is great because lipopolysaccharide (LPS) is highly toxic. For example, the LD₅₀ for the lipopolysaccharide in sheep is about 10-20 µg/kg (intravenous), while in mice it is about 5 mg/kg. In sheep (and probably also in humans) lipopolysaccharide causes death by releasing mediators that trigger pulmonary hypertension, pulmonary edema, and peripheral vascular collapse. Death usually occurs within 8 to 48 hours after injection of lipopolysacoharide or lipid A. Occasionally, death will occur at 1-2 weeks. This is usually the result of disseminated intravascular coagulopathy leading to renal cortical necrosis and uremic death.

The pretreatment of mammals, such as sheep or mice, with a compound identified as a GTPase inhibitor by the assay of the present invention should make the mammal resistant to the lethal effects of Gram-negative endotoxin. We also envision that treatment of a mammal after the symptoms of endotoxin shock appear will lessen disease symptoms. Of note, current therapies are aimed at killing the gram negative bacteria (antibiotics) and at neutralizing circulating endotoxin (anti-endotoxin antibodies). However, these therapies have the disadvantages of releasing more endotoxin and having no effect on endotoxin already internalized into cells. This last disadvantage is clinically very relevant because the signs and symptoms of endotoxemia are often not manifest until 1.5-3 hours after the endotoxin is released into the patient, thus rendering anti-endotoxin antibodies less effective. The apparent antagonism between the compounds identified as GTPase inhibitors and endotoxin can have useful applications in clinical situations and disease states that are caused by endotoxin, such as Gram-negative sepsis following surgery in humans and animals, bovine or porcine mastitis, and other endotoxin-related veterinary diseases listed in Tables I and II.

While LPS activation of macrophage-purinoreptors is a specific case of macrophage activation, this may represent a more general case for macrophage activation. For example, endotoxin (LPS) may substitute for naturally occurring mammalian lipids that are released during tissue damage along with intracellular ATP or ADP. This combination of lipid plus purine might then activate macrophages. Hence, 2-MeSATP and other purino-receptor-active derivatives might block macrophage activation seen in a number of other pathophysiological conditions. Thus, several other disease states wherein 2-MeSATP might be useful are listed in Table IV.

We have shown that lipid X can prevent the detrimental effects of LPS and that lipid X, like 2-methylthio-ATP, inhibits GTPase activity in the assay of the present invention. In addition to 2-methylthio-ATP and lipid X, there are other compounds that are likely to possess the ability to protect against the effect of Gram-negative endotoxin. Representative compounds include ATP analogs with R groups at the 2 and 8 positions of the nitrogen ring. (2-MeSATP has a -SCH₃ group at the 2 position of the nitrogen ring.) Useful R groups include -OCH₃, -NHCH₃, -CH₂CH₃, -CH₃, -CH=CH₂, -COOH, -OH, -SH, -NH₂, and longer and/or branched aliphatic chains attached directly to the ring or through -O, -N, or -S linkage, as well as through various ester and amide bonds. Halogenated compounds, such as 2-Chloro-ATP, might also function similarily to 2-MeSATP. For a compound to be effective, it must interact with the purinoreceptor system and be a GTPase antagonist.

Preliminary results suggest that compounds such as β, γ-Methylene-ATP, with carbon substituted for oxygen in the phosphate linkage, may be useful. We also envision that derivatives of the adenine nitrogen ring might be useful. We have examined the effects of IBMX, NAD, AMP, UTP, theophylline, and adenosine (at 100µM). These adenine-like purines and a pyrimidine did not synergistically increase the LPS-induced GTPase activity. In that respect; they are similar to 2-MeSATP.

### b. Specific Animals Tested

In the following two experiments with sheep and mice, we sought to measure the ability of lipid X, which inhibit LPS-induced GTPase activity in vitro, to inhibit entoxin shock in vivo.

Mixed breed sheep weighing 30-70 kg were obtained from the University of Wisconsin Experimental Farms. Sheep were prepared as follows: at least 24 hours before each experiment, catheters were percutaneously placed in the pulmonary artery. Any other catheterizations that were required for infusion were done at this time. All studies were performed on awake, unrestrained sheep placed in portable stanchions with food and water ad libitum. Electronically calculated pulmonary artery pressures as well as heart rates were recorded at various intervals on a polygraph. To ensure a stable baseline prior to the injection of test compounds, approximately 1 hour of baseline measurements were recorded.

Eight week old mice (strain C57BL/10) were allowed to rest for at least 7 days following their arrival and were sexually segregated and housed at 5 per cage with food and water ad libitum. Intravenous injection of a GTPase inhibiting compound (lipid X dissolved in saline as described above) and/or lipopolysaccharide was achieved by the retroorbital sinus route using a 27 gauge needle. No more than 0.1 ml of fluid was injected at a time. Just prior to injection, the mice were lightly anesthetized with ether. Following the injection, mice were observed every 0.5-6 hours and the time of death was recorded. In a few experiments, mice were injected by the intraperitoneal route with solutions of lipid X and/or lipopolysaccharide similar to those described above.

Protection of mice against lethal endotoxemia.

To test for the toxicity of lipid X in mice, C57BL/10 mice were challenged with 750, 2000 or 5000 µg of lipid X intraperitoneally (12 mice) or with 750, 1500 or 3000 µg intravenously (7 mice). All these mice lived. Consequently, lipid X appeared to be nontoxic in mice, as in sheep. As discussed more fully below, 40 sheep have been injected with lipid X and no serious adverse complications of lipid X administration have been observed so far.

The lethal dose of E. coli endotoxin was determined both for the intravenous and for the intraperitoneal challenge. The lethal dose that killed 100% of the mice (LD₁₀₀) was 250 µg intravenously and 500 µg intraperi toneally. (It is important to standardize each lot of endotoxin with each lot of mice.) To determine the approximate dose of lipid X needed to protect against a lethal challenge of endotoxin, mice were pretreated with lipid X intraperitoneally 2 hours before challenge with 1500 µg of endotoxin, which is 3 times the LD₁₀₀ dose. Pretreatment of mice with lipid X appeared to prolong the time to death four-fold (from about 20 hours to 80 hours).

Because both lipid X and lipopolysaccharide (LPS) were given intraperitoneally in these preliminary experiments, the possibility existed of slow or variable rates of absorption. Consequently, an intravenous challenge route was selected to test for protective efficiency. Mice were given 750 µg of lipid X from 24 hours prior to 6 hours after endotoxin challenge with 1-2 LD₁₀₀ doses. Forty-one percent of mice receiving 500 µg lipid X from 6 hours prior to endotoxin challenge through 1 hour after LPS challenge survived. When a lower endotoxin challenge dose (250 µg) was used, 81% of the mice survived if treated with lipid X from 1 hour prior to through 4 hours post LPS challenge. At both challenge doses of endotoxin, the group of mice which received lipid X shortly after endotoxin challenge showed higher mortality than the group on either side. Perhaps this is due to the fact that those animals were anesthetized twice in a short period and received the toxic endotoxin challenge before receiving lipid X. The reversal of the lethal toxicity of endotoxin at times as late as 4 hours after endotoxin challenge is especially striking. Although this was best demonstrated at the lower endotoxin dose of 250 µg per mouse, this still represents a massive challenge (approximately 10 mg of lipopolysaccharide per Kg body weight). By 4 hours after injection of the 250 µg of intravenous lipopolysaccharide, the mice had stopped normal behavior, i.e. nesting had stopped, decreased spontaneous activity occurred, and the animals were shaking.

Protection of sheep against lethal endotoxemia. As noted above, the LD₅₀ of lipopolysaccharide in sheep is in the vicinity of 10-20 µg/kg, injected intravenously. Sheep respond to lipopolysaccharide in a manner that more closely resembles the situation encountered in human diseases.

As reported in the literature, lipid X by itself (40-1000 µg/kg) does not create marked illness or fever, only a period of transient pulmonary hypertension and slight shortness of breath subsides after 30 minutes. About 30 sheep were examined by Burhop with regard to lipid X toxicity.

In contrast to lipid X, a single injection of LPS (10-20 µg/kg) causes serious pulmonary hypertension, and after 15-30 minutes, an animal treated with LPS will begin to tremble, cough and lay down. The symptoms become more severe over the next few hours and are accompanied by fever. About half the animals die in 24 hours.

In view of the partial protection afforded by lipid X against LPS-induced lethality in mice, the possibility of such protection was also examined in sheep. The results are summarized in Table III. The sheep system has the important advantage of permitting the administration of much larger doses of lipid X relative to LPS. It is the ratio of lipid X to endotoxin that is critical for survival. As shown in Table III, all the sheep receiving 100-200 µg/kg of lipid X during the pretreatment period survived subsequent LPS challenge. It is evident that a 5-10 fold excess of lipid X (on a weight basis) over LPS is required to ensure survival. (Because of the inherent LPS resistance of mice, their small body volume, and the limited solubility of lipid X in water, it is impossible to achieve 5-10 fold excess of lipid X relative to LPS in the mouse system.)

In addition to rescuing sheep from lethal effects of LPS, pretreatment with lipid X also alleviates some of the serious clinical symptoms, including shortness of breath, weakness, diarrhea, etc. However, lipid X does not completely prevent pulmonary hypertension or fever induced by LPS.

Previous work on the lethal endotoxicity of LPS showed that only limited prevention of the complications of injection of this material could be achieved through the administration of glucocorticoids, prostaglandins, naloxone, pressors, fluid replacement therapy or anti-LPS antibodies. In addition, all existing therapies against LPS lethality are dependent upon their being given prior to or very shortly after the administration of the LPS challenge. However, in the mouse lipid X appears to prevent lethal endotoxicity even when given 6 hours after lipopolysaccharide.

The acute protection afforded by lipid X may have some relationship to the biological phenomenon of LPS tolerance described in the clinical literature. However, lipid X-induced protection against LPS is immediate and wears off after several hours, whereas classical tolerance does not appear for many days and may, in part, be mediated by antibodies.

The invention is useful to ameliorate pathological conditions created by many of the endotoxin-induced diseases listed in Table I.
Furthermore, protection with such compounds might be obtained even after the signs and symptoms of endotoxemia had been developed. This is an extremely important therapeutic consideration, since the signs and symptoms of a disease must almost always manifest before therapy is initiated. Although the mechanism(s) by which protective compounds inhibit LPS-inducable GTPase activity in the assay remain unknown, the data fit best for agents interupting a signal transduction pathway involving purinoreceptors that are linked to an endotoxin-responsive G-protein (i.e., GTPase) that ultimately leads to the release of the TNF by macrophages.

The compounds identified as inhibiting GTPase activity and their various modified derivatives may be introduced into the circulation of an animal by intravenous, intraperitoneal or intramuscular routes, to induce a state of relative resistance to the deleterious effect of lipopolysaccharide. When thus employed, the compounds may be administered in the form of parenteral solutions containing the selected protective compound, in a sterile liquid suitable for intravenous or other administration. The exact route, dose, and administration interval of the active compound will vary with the size and weight of the animal, and the species, and the desired level of protection. However, in general the dosage will be similar to that for lipid X and proportional to the ability of the compound to inhibit GTPase as compared to lipid X.

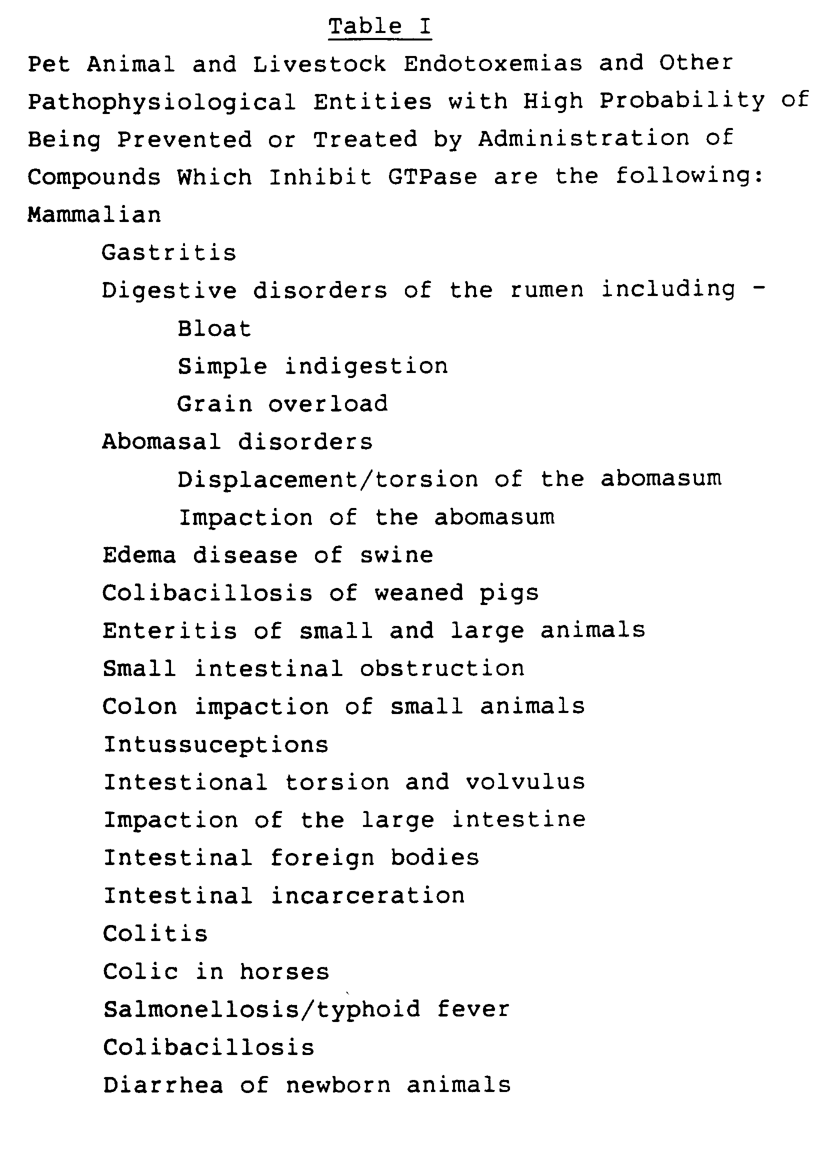

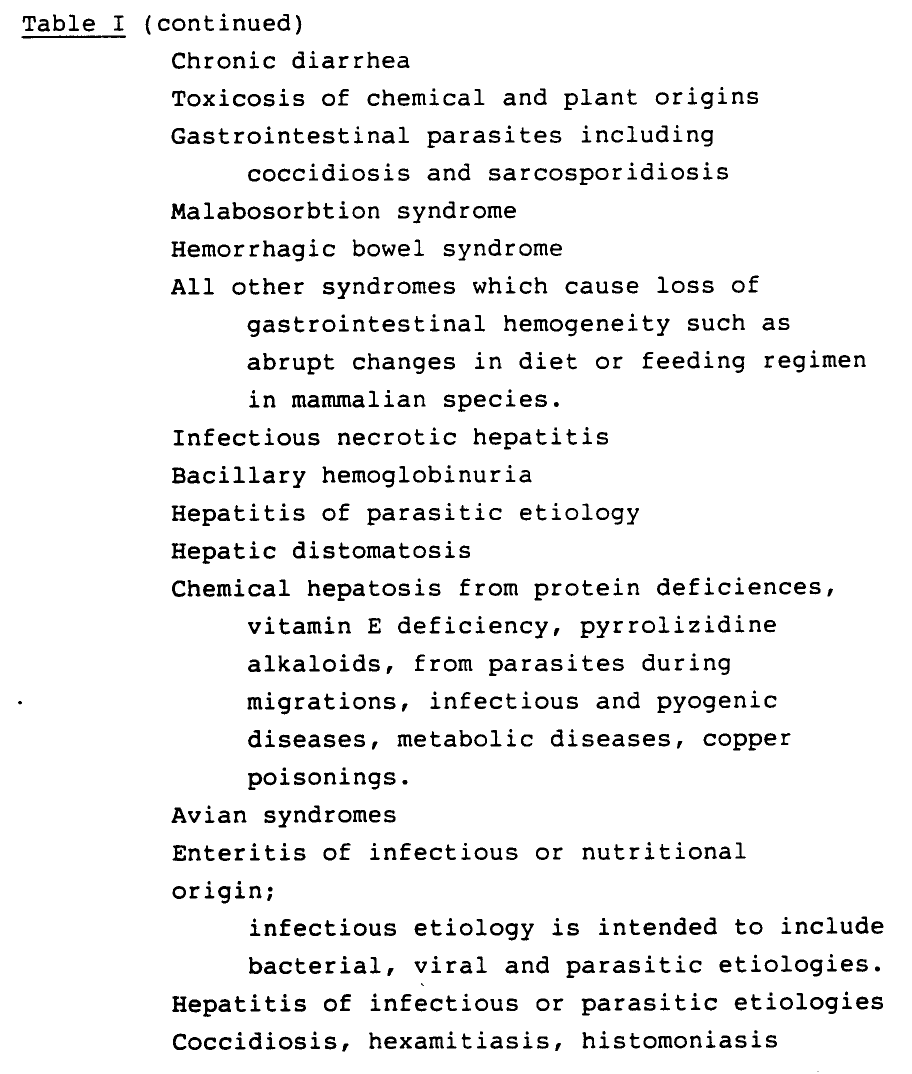

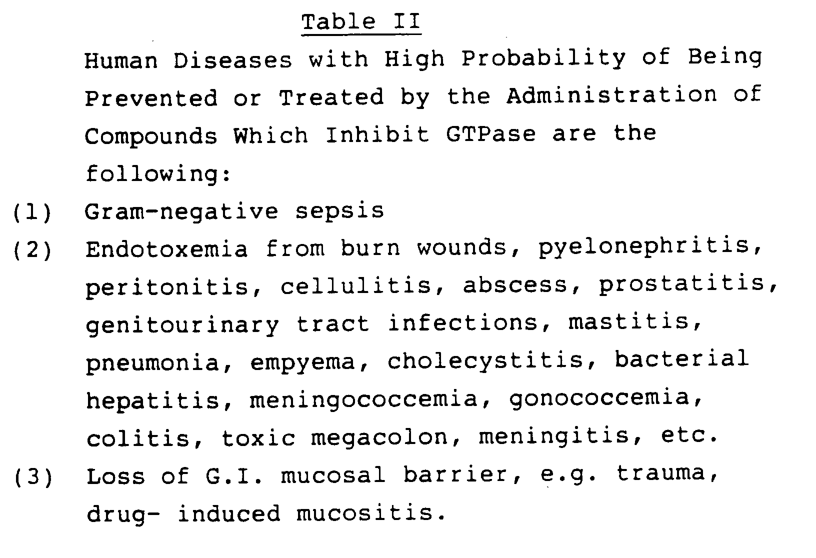

**Table III**

| Protection of Sheep Against the Lethal Effects of Intravenous Lipopolysaccharide | | | |
|---|---|---|---|
| Number of Sheep | Dose of Lipid X (µg/kg) | Dose of Lipopolysaccharide (µg/kg) | Survivors |
| 3 | 0 | 10 | 2/3 |
| 8 | 0 | 20 | 4/8 |
| 3 | 200^{a)} | 20 | 3/3 |
| 3 | 100^{b)} | 10 | 3/3 |
| 4 | 100^{a)} | 20 | 4/4 |
| 2 | 50^{a)} | 20 | 1/2 |

| | | | |
|---|---|---|---|
| ^{a)} Administered 1 hour prior to endotoxin. | | | |
| ^{b)} Administered 15 min. prior to endotoxin. | | | |

**Table IV**

| Other non-endotoxin uses for purinoreceptor-inhibitors: |
|---|
| Pancreatitic |
| Myocardial infarction |
| Crush Trauma |
| Burns (chemical and thermal) |
| Vasculitis |
| Drug toxicities involving macrophage activation |
| Toxic shock syndrome |
| Enterotoxin |
| Overwhelming Viremia |
| Viral pneumonia |
| Immune complex diseases |
| Aspiration pneumonia |
| Drowning |
| Inhaled toxins or irritants |
| Shock lung |
| Reperfusion injuries |

## Claims

1. Use of a compound which inhibits lipopolysaccharide-induced (LPS-induced) GTPase, excluding lipid X, for the preparation of a pharmaceutical composition for the prevention of the detrimental effects of endotoxin in a mammal.

2. Use of 2-methylthio-ATP for the preparation of a pharmaceutical composition for the prevention of the detrimental effects of endotoxin in a mammal.

3. Use of a compound which inhibits LPS-induced GTPase, excluding lipid X, for the preparation of a pharmaceutical composition for the prevention of the detrimental effects of endotoxin shock mediators in a mammal.

4. Use of 2-methylthio-ATP for the preparation of a pharmaceutical composition for the prevention of the detrimental effects of endotoxin shock mediators in a mammal.

5. Use of claim 3 or claim 4, wherein the endotoxin shock mediator is tumor necrosis factor (TNF).

6. An assay for identifying compounds which will protect mammals from the toxic effects of Gram-negative endotoxin, which comprises measuring the ability of said compounds to inhibit LPS-induced GTPase which is a measurement of their usefulness in protecting mammals.

7. An assay for identifying if a candidate compound will protect mammals from the toxic effects of Gram-negative endotoxin, which comprises adding a candidate compound to a reaction buffer containing HEPES (ph 7.4); ATP or ADP; LPS; macrophage membranes; and γ-³²P-GTP, incubating the reaction buffer at 30°C or 37°C for approximately 15 to 30 minutes, and then measuring the degree inhibition of LPS-induced GTPase inhibition by measuring the amount of γ-³²Pi released.

## Patentansprüche

1. Verwendung einer Verbindung, die durch Lipopolysaccharid induzierte (LPS-induzierte) GTPase inhibiert, unter Ausschluß von Lipid X, zur Herstellung einer pharmazeutischen Zusammensetzung zur Verhütung der schädlichen Auswirkungen von Endotoxin in einem Säuger.

2. Verwendung von 2-Methylthio-ATP zur Herstellung einer pharmazeutischen Zusammensetzung zur Verhütung der schädlichen Auswirkungen von Endotoxin in einem Säuger.

3. Verwendung einer Verbindung, die LPS-induzierte GTPase inhibiert, unter Ausschluß von Lipid X, zur Herstellung einer pharmazeutischen Zusammensetzung zur Verhütung der schädlichen Auswirkungen von Endotoxinschockmediatoren in einem Säuger.

4. Verwendung von 2-Methylthio-ATP zur Herstellung einer pharmazeutischen Zusammensetzung zur Verhütung der schädlichen Auswirkungen von Endotoxinschockmediatoren in einem Säuger.

5. Verwendung nach Anspruch 3 oder Anspruch 4, worin der Endotoxinschockmediator der Tumornekrosefaktor (TNF) ist.

6. Assay zur Identifizierung von Verbindungen, die Säuger vor den toxischen Effekten von gramnegativem Endotoxin schützen, das umfaßt Messen der Fähigkeit dieser Verbindungen, LPS-induzierte GTPase zu inhibieren, die ein Maß für ihre Nützlichkeit zum Schutz von Säugern ist.

7. Assay zur Identifizierung, ob eine Testverbindung Säuger vor den toxischen Effekten von gramnegativem Endotoxin schützt, das umfaßt Zugeben einer Testverbindung zu einem Reaktionspuffer, der HEPES (pH 7,4), ATP oder ADP, LPS, Makrophagenmembranen und γ-³²P-GTP enthält, Inkubieren des Reaktionspuffers ungefähr 15 bis 30 Minuten lang bei 30 °C oder 37 °C und dann Messen des Inhibierungsgrads der LPS-induzierten GTPase-Inhibierung durch Messen der Menge an freigesetztem γ-³²Pi.

## Revendications

1. Utilisation d'un composé qui inhibe l'activation de la GTP-ase par les lipopolysaccharides (activation par LPS), à l'exclusion du lipide X, pour la préparation d'une composition pharmaceutique destinée à la prévention des effets nocifs d'endotoxine chez un mammifère.

2. Utilisation de 2-méthylthio-ATP pour la préparation d'une composition pharmaceutique destinée à la prévention des effets nocifs d'endotoxine chez un mammifère.

3. Utilisation d'un composé qui inhibe l'activation de la GTP-ase par LPS, à l'exclusion du lipide X, pour la préparation d'une composition pharmaceutique destinée à la prévention des effets nocifs des médiateurs de choc endotoxinique chez un mammifère.

4. Utilisation de 2-méthylthio-ATP pour la préparation d'une composition pharmaceutique destinée à la prévention des effets nocifs des médiateurs de choc endotoxinique chez un mammifère.

5. Utilisation selon la revendication 3 ou la revendication 4, dans laquelle le médiateur de choc endotoxinique est le facteur de nécrose tumorale (TNF).

6. Dosage pour identifier des composés assurant la protection de mammifères contre les effets toxiques d'endotoxine de micro-organismes Gram-négatifs, qui comprend la mesure de la capacité desdits composés à inhiber l'activation de la GTP-ase par LPS afin d'évaluer leur utilité dans la protection des mammifères.

7. Dosage pour déterminer si un composé potentiel protégera des mammifères des effets toxiques d'endotoxine de micro-organismes Gram-négatifs, qui comprend l'addition d'un composé potentiel à un tampon réactionnel contenant HEPES (pH 7,4); ATP ou ADP; LPS; des membranes de macrophages; et γ-³²P-GTP, la mise en incubation du tampon réactionnel à 30°C ou 37°C pendant environ 15 à 30 minutes, et la mesure du degré d'inhibition de l'activation de GTP-ase par LPS en dosant la quantité de γ-³²P libérée.
